# EUROPEAN PATENT APPLICATION

(11) **EP 1 997 507 A2**
(43) Date of publication of application: **03.12.2008**
(21) Application number: 08290361.8
(22) Date of filing: 11.04.2008
(51) Int. Cl.: A61K 38/18, A61P 19/00

(54) **Treatment of annulus fibrosis defects**

(30) Priority: 11.04.2007 US 786154
(71) Applicant: Ebi, L.P., NJ New Jersey Parslppany 07054 (US)
(72) Inventor: Simon, Bruce, Mountain Lakes New Jersey 07046 (US); Simon, Joshua, Rockaway New Jersey 07866-1920 (US)
(74) Representative: Hirsch & Associés

(57) **Abstract**

Compositions and methods for treating an annulus fibrosis defect comprising an angiogenic factor and a carrier. Methods include administering the composition to the annulus fibrosis defect, where the administering can include injecting the composition. The present compositions can stimulate angiogenesis and revascularization thereby facilitating repair of the defect.

## Description

### INTRODUCTION

The present technology relates to compositions and methods for treating tears, fissures, or openings in the annulus fibrosis of an intervertebral disc, for example, such as those resulting from degenerative disc disease or discectomy.

Intervertebral discs of the spinal column are composed of the nucleus pulposus and the annulus fibrosis with the cartilaginous endplate acting as the interface between the rest of a disc and the vertebral body. The intervertebral disc contains substantial portions of water, collagen, and proteoglycans. Strong fibers of the outer annulus fibrosis surround and contain the inner nucleus pulposus in order to distribute pressure evenly about the disc and provide tensile strength. The inner nucleus pulposus contains a higher content of proteoglycans than the annulus fibrosis and has a gel-like consistency which provides compressive strength.

Concentric and/or transverse tears in the annulus fibrosis are not uncommon. Development of a radial tear extending to the disc nucleus is a hallmark of disc degeneration. Herniation of both nuclear material and the annulus fibrosis may occur through the tear, compromising the effective shock absorbing capacity of the disc, and may result in considerable pain.

Methods for treating disc fissures, tears, and associated pain may include a discectomy, which is the surgical removal of a herniated segment of the intervertebral disc. However, a hole following discectomy or a tear or fissure in the annulus may lead to additional complications and further degeneration of the disc. In some instances, degenerative discs of a severe nature are treated with spine fusion surgery.

Effective compositions and methods to treat annular fibrosis defects are desirable. Such treatments would provide alternatives to more invasive methods, such as vertebral fusion or disc replacement using a partial or fully artificial disc. Moreover, treatments that complement the body's natural healing processes are preferable to repeated surgical interventions and/or replacement of intervertebral discs with artificial or partially artificial discs.

### SUMMARY

The present technology provides compositions and methods for treating annulus fibrosis defects in an intervertebral disc. The compositions can comprise an angiogenic factor and a carrier. Angiogenic factors useful herein include those of the formula 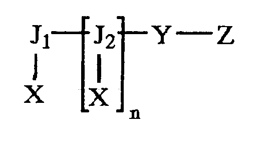 wherein each X is a synthetic peptide chain that binds an FGF receptor and has from 3 to about 50 amino acid residues; n is 0 or 1; J¹ is an amino acid; J² is a diamino acid when n=1, or is absent when n=0; Y is a linker that comprises a chain of from about 9 to about 50 atoms, is not found in the natural ligand of the FGF receptor, and is covalently bonded to J¹ and Z when n=0, or to J² and Z when n=1; Z represents a non-signaling peptide that comprises a heparin binding domain, comprising an amino acid sequence that comprises from 1 to about 10 heparin binding motifs and a maximum of about thirty amino acids; wherein when n=1 the synthetic peptide chains, X, are identical; and wherein the peptide analog has an avidity for heparin such that the synthetic heparin-binding growth factor analog binds heparin in 0.48M NaCl, but is eluted by 1 M NaCl. Carriers include various materials that form a viscous or gel-like solution.

The present technology also provides uses of compositions for the manufacture of medicaments for the treatment of an annulus fibrosis defect, such compositions comprising an angiogenic factor and a carrier. Treatment can include applying the composition during a surgical procedure such as a discectomy. The composition may be injected using a syringe during surgery or as a non-surgical treatment affording a minimally invasive treatment method.

The present technology affords benefits, such as one or more of promoting and initiating angiogenesis at the site of an annulus fibrosis defect, vascularizing the defect, and re-vascularizing defect. Compositions and methods of the present technology can thereby heal, enhance healing, or provide more complete healing of annulus fibrosis defects such as tears, fissures, or openings left following a discectomy.

### DESCRIPTION

The following description of technology is merely exemplary in nature of the subject matter, manufacture and use of one or more inventions, and is not intended to limit the scope, application, or uses of any specific invention claimed in this application or in such other applications as may be filed claiming priority to this application, or patents issuing therefrom. The following definitions and non-limiting guidelines must be considered in reviewing the description of the technology set forth herein.

The headings (such as "Introduction" and "Summary") and subheadings (such as "Carriers") used herein are intended only for general organization of topics within the disclosure of this technology, and are not intended to limit the disclosure of the technology or any aspect thereof. In particular, subject matter disclosed in the "Introduction" may include novel technology, and may not constitute a recitation of prior art. Subject matter disclosed in the "Summary" is not an exhaustive or complete disclosure of the entire scope of the technology or any embodiments thereof.

The citation of references herein and during prosecution of patent applications regarding this technology does not constitute an admission that those references are prior art or have any relevance to the patentability of the technology disclosed herein. All references cited in the Description section of this specification are hereby incorporated by reference in their entirety.

The description and specific examples, while indicating embodiments of the technology, are intended for purposes of illustration only and are not intended to limit the scope of the technology. Moreover, recitation of multiple embodiments having stated features is not intended to exclude other embodiments having additional features, or other embodiments incorporating different combinations of the stated features. Specific Examples are provided for illustrative purposes of how to make, use and practice the compositions and methods of this technology and, unless explicitly stated otherwise, are not intended to be a representation that given embodiments of this technology have, or have not, been made or tested.

As used herein, the words "preferred" and "preferably" refer to embodiments that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the technology.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that a recitation that an embodiment can or may comprise certain elements or features does not exclude other embodiments of the present technology that do not contain these elements or features.

The present technology involves the treatment of tissue defects in humans or other animal subjects. Specific materials to be used in the technology must, accordingly, be biomedically acceptable and biocompatible. As used herein, such a "biomedically acceptable" component is one that is suitable for use with humans and/or animals without undue adverse side effects (such as toxicity, irritation, and allergic response) commensurate with a reasonable benefit/risk ratio. As used herein, such a "biocompatible" component is one that is suitable for use with humans and/or animals without undue adverse side effects (such as toxicity, irritation, and allergic response) commensurate with a reasonable benefit/risk ratio.

### Angiogenic Factor:

The present invention provides various compositions useful for treating annulus fibrosis defects, comprising an angiogenic factor and a carrier. In some embodiments, the angiogenic factor includes a synthetic angiogenic factor having the formula (1): 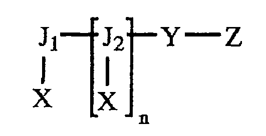 wherein each X is a synthetic peptide chain that binds an FGF receptor and has from 3 to about 50 amino acid residues, n is 0 or 1; J¹ is an amino acid; J² is a diamino acid when n=1, or is absent when n=0; Y is a linker that comprises a chain of from about 9 to about 50 atoms, is not found in the natural ligand of the FGF receptor, and is covalently bonded to J¹ and Z when n=0, or to J² and Z when n=1; Z represents a non-signaling peptide that comprises a heparin binding domain, comprising an amino acid sequence that comprises from 1 to about 10 heparin binding motifs, and a maximum of about thirty amino acids; wherein when n=1 the synthetic peptide chains, X, are identical; and wherein the peptide analog has an avidity for heparin such that the synthetic heparin-binding growth factor analog binds heparin in 0.48M NaCl, but is eluted by 1M NaCl. Angiogenic factors having formula (1) are disclosed in U.S. Patent Publication No. 2004/0038348, Pena at al., published February 26, 2004.

The X, Y and Z moieties of formula (1) include amino acid residues, of the formula -NHRCO-, where R can be hydrogen or any organic group. The amino acids can be D-amino acids or L-amino acids. The amino acids (1) can include any of the twenty amino acids found naturally in proteins, as well as any of the naturally occurring amino acids not found naturally in proteins.

In formula (1), when n is 0, the molecule includes a single X moiety and the molecule is a linear chain. When n is 1 in formula (1), the molecule includes two X moieties that are identical in amino acid sequence. In the latter case the molecule is a branched chain that may also be constrained by cross-links between the two X moieties as described below. In this embodiment, each molecule can bind two heparin binding growth factor receptors (HBGFRs) and induce receptor dimerization. Advantageously, the dimerization in turn potentiates enhanced receptor signaling activity of the HBGFRs.

When n is 0 in formula (1), the X region is covalently linked through an amino acid, J¹ to the hydrophobic region Y. When n is 1 in formula (1), one X moiety is covalently linked through an amino acid J¹, which is in turn covalently linked to a second amino acid, J², which is a diamino acid. J¹ is linked to one amino group of the diamino acid, J². The second X moiety is covalently linked to J² through the second amino group of the diamino acid. J² is then covalently linked through its carboxy terminus to the Y moiety of the molecule. The amino acid J¹ of formula (1) can be any of the amino acids described above. The diamino acid J² of formula (1) can be any diamino acid, such as for instance lysine, or ornithine, or any other amino acid having two amino groups.

The X moiety of formula (1) is a synthetic peptide chain that binds an HBGFR. X can, for example, have any amino acid sequence that binds an HBGFR, and can include amino acid sequences that are identical to a portion of the amino acid sequence of an HBGF. Various embodiments of the present technology include the angiogenic factor of formula (1) wherein X comprises an amino acid sequence found in any of FGF-1, FGF-2, FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF 22, and FGF-23. Alternatively, X can have an amino acid sequence homologous rather than identical to the amino acid sequence of a heparin binding growth factor (HBGF). The particular HBGFR bound by the molecule of formula (1) may or may not be the cognate receptor of the original HBGF, i.e. the molecule of formula (1) may additionally or solely bind to the receptor of a different HBGF. In various embodiments, the amino acid sequences of the X moieties of formula (1) include homologs of fragments of naturally occurring HBGFs that differ from the amino acid sequences of natural growth factor in only one or two or a very few positions. In another alternative, X can include an amino acid sequence that shows no detectable homology to the amino acid sequence of any HBGF. X may comprise from 3 to 50, from 6 to 40, or from 10 to 30 amino acid residues.

In one embodiment, an angiogenic factor of formula (1) includes two X moieties, where the X moieties are covalently cross linked. Suitable cross links can be formed by disulfide S-S bridges of cysteines linking the two X moieties. Alternatively, the cross link can be conveniently formed during simultaneous and parallel peptide synthesis of the X moiety amino acids chains by incorporating a lanthionine (thio-dialanine) residue to link the two identical X chains at alanine residues that are covalently bonded together by a thioether bond. In another method the two X moiety amino acid chains can be cross-linked by introducing a cross-linking agent, such as a dicarboxylic acid, e.g. suberic acid (octanedioic acid), or the like, thereby introducing a hydrocarbon bridge between the two identical X moieties having a free amino, hydroxyl or thiol group.

The Y moiety of formula (1) represents a linker that is sufficiently hydrophobic to non-covalently bind the HBGF analog to a polystyrene or polycaprolactone surface, or the like. In addition, Y may bind to other hydrophobic surfaces, particularly the hydrophobic surfaces formed from materials used in medical devices. The amino acid sequence of Y is an artificial sequence, i.e., it does not include any amino acid sequence of four or more amino acid residues found in a natural ligand of an HBGF.

The Y moiety of formula (1) includes a chain of atoms or a combination of atoms that form a chain. Typically, the chains comprise carbon atoms, and may also optionally include oxygen, nitrogen or sulfur atoms, such as for example chains of atoms formed from amino acids (e.g. amino acids found in proteins, as listed above; naturally occurring amino acids not found in proteins, such as ornithine and citrulline; or non natural amino acids, such as amino hexanoic acid; or a combination of any of the foregoing amino acids). The chain of atoms of the Y moiety is covalently attached to J¹ or J², and to peptide Z. The covalent bonds can be, for example, amide or ester bonds. In various embodiments, Y includes a chain of from 9 to 50,12 to 45, or 15 to 35 atoms. For example, Y may be formed from a chain of from 4 to 17, 5 to 15, or 6 to 12 amino acids. Alternatively, Y may be formed from a chain of at least one, at least two, or at least three aminohexanoic acid residues.

In various embodiments, the Y moiety can include a branched or unbranched, saturated or unsaturated alkyl chain of between one and about twenty carbon atoms. In a further embodiment, Y can include a chain of hydrophobic residues, such as for instance, ethylene glycol residues, or a chain of hydrophobic amino acid residues.

The Z moiety is a heparin-binding region and can include one or more heparin-binding motifs, BBxB or BBBxxB, such as described by Verrecchio et al. J.Biol.Chem. 275: 7701, (2000). Alternatively, Z can include both BBxB and BBBxxB motifs (where B represents lysine, arginine, or histidine, and x represents a naturally occurring, or a non-naturally occurring amino acid). For example, the heparin-binding motifs may be represented by the sequence [KR][KR][KR]X(2)[KR], designating the first three amino acids as each independently selected from lysine or arginine, followed by any two amino acids and a fifth amino acid which is lysine or arginine.

The number of heparin binding motifs is not critical. For instance, Z may include at least one, at least two, at least three or at least five heparin-binding motifs, up to a maximum of about ten heparin-binding motifs. In another alternative embodiment, the Z region includes from 4 to 30, 6 to 25, or 8 to 20 amino acid residues.

In a preferred embodiment, the amino acid sequence of the Z moiety is RKRKLERIAR. Heparin-binding domains that bear little or no sequence homology to known heparin-binding domains are also useful. "Heparin-binding" also includes binding to the -NHSO₃⁻ and sulfate modified polysaccharide, heparin and also binding to the related modified polysaccharide, heparan.

The Z moiety confers the property of binding to heparin in low salt concentrations, up to about 0.48M NaCl, forming a complex between heparin and the Z region of the factor analog. The complex can be dissociated in 1M NaCl to release the synthetic HBGF analog from the heparin complex. The Z moiety is a non-signaling peptide. Accordingly, when used alone the Z moiety binds to heparin which can be bound to a receptor of a HBGF, but the binding of the Z moiety peptide alone does not initiate or block signaling by the receptor. In a preferred embodiment the C terminus of the Z moiety is an amidated arginine.

In some embodiments, the angiogenic factor is the synthetic peptide F2A4-K-NS, which is a species of the molecule of formula (1) as described by Lin X et al., Synthetic peptide F2A4-K-NS mimics fibroblast growth factor-2 *in vitro* and is angiogenic *in vivo,* Int'l J Mol. Med. 17:833-839 (2006). F2A4-K-NS is a branched peptide with the following amino acid sequence: H-YRSRKYSSWYVALKRK(H-YRSRKYSSWYVALKR)-Ahx-Ahx-Ahx-RKRLDRIAR-NH₂, wherein Ahx represents aminohexanoic acid. The amino acid sequence YRSRKYSSWYVALKR, of the two X region peptides corresponds to amino acids 115-129 of FGF-2 identified by Ray et al., A 10-amino acid sequence of fibroblast growth factor 2 is sufficient for its mitogenic activity on neural progenitor cells, Proc. Natl. Acad. Sci. USA 94:7047-7052 (1997).

### Carrier:

In some embodiments, carriers or vehicles for the angiogenic factors include any viscous gel. The viscous gel should have the mechanical properties of a hydrogel, which help retain the one or more angiogenic factors locally at the site of injection or application by prohibiting diffusion. Carriers can include natural molecules, chemically modified natural molecules, mechanically processed natural molecules, synthetic polymers, and mixtures thereof. Carriers can include various aqueous solutions which can contain other solvents, such as organic solvents. Carriers can further comprise various buffers and salts to adapt the gel to physiological conditions, including pH and osmotic pressure.

Suitable carriers can include one or more gel materials selected from collagen, keratin, chitosan, laminin, fibronectin, vitronectin, poly-D-lysine, Matrigel^{™} (BD Biosciences, San Jose, CA), PuraMatrix^{™} (BD Biosciences, San Jose, CA), Cell-Tak^{™} (BD Biosciences, San Jose, CA), agarose, cellulose, polyethylene glycol, polypropylene glycol, poly(2-acrylamido-2-methyl-1-propanesulfonic acid), polyvinyl pyrrolidone, silicone hydrogel, glycosaminoglycan and polyacrylamide. In various embodiments, the carrier is a glycosaminoglycan selected from the group consisting of chondroitin sulphate, dermatan sulphate, keratan sulphate, heparan sulphate, heparin, and hyaluronan (hyaluronic acid).

Viscosity of the angiogenic factor and carrier mixture can be adjusted depending on the intended site and method of administration. For example, formulations with increased concentrations of carrier can be used in applications where additional reduction in the diffusion of the angiogenic factor is desired. Alternatively, the carrier consistency and viscosity can be reduced to make the composition more fluid for applications where the composition is to be injected, which can depend on the gauge of the syringe needle to be employed and the volume of material to be dispensed. Viscosity measurements and formulations of the present compositions are readily determined and these parameters can be adapted as needed.

The compositions may also comprise one or more bioactive materials that provide a therapeutic or nutritional benefit for the human or other animal subject in which the composition is injected or applied, including maintaining, improving or otherwise affecting the structure or function of tissue proximate to the defect site. In various embodiments, such benefits include one or more of repairing of unhealthy or damaged tissue, minimizing infection at the implant site, increasing integration of healthy tissue into the defect site, and preventing disease or defects in healthy or damaged tissue.

If present, a bioactive material is preferably included at a safe and effective amount. A "safe and effective" amount of bioactive material is an amount that is sufficient to have the desired effect in the human or lower animal subject, without undue adverse side effects (such as toxicity, irritation, or allergic response), commensurate with a reasonable benefit/risk ratio when used in the manner of this invention. The specific safe and effective amount of the bioactive material will, obviously, vary with such factors as the particular condition being treated, the physical condition of the patient, the nature of concurrent therapy (if any), the specific bioactive material used, the specific route of administration and dosage form, the carrier employed, and the desired dosage regimen.

Bioactive materials useful herein include organic molecules such as proteins, peptides, peptidomimetics, nucleic acids, nucleoproteins, antisense molecules polysaccharides, glycoproteins, lipoproteins, carbohydrates and polysaccharides, and synthetic and biologically engineered analogs thereof; living cells such as chondrocytes, bone marrow cells, and stem cells; viruses and virus particles; natural extracts; and combinations thereof. Specific examples of bioactive materials include hormones, antibiotics, antivirals, and other antiinfective agents, hematopoietics, thrombopoietics, antitumoral agents (chemotherapeutic agents), antipyretics, analgesics, antiinflammatory agents, antiallergic agents, anticoagulants, therapeutic agents for osteoporosis, enzymes, vaccines, immunological agents and adjuvants, cytokines, growth factors, cellular attractants and attachment agents, gene regulators, vitamins, minerals and other nutritionals, and combinations thereof.

In some embodiments, a composition of the present technology is loaded into a syringe prior to use. Preloaded syringes can include one or more preservatives to stabilize the composition and prevent degradation of the angiogenic factor and carrier during storage.

Syringes containing the present compositions can be fitted with or have an integral hypodermic needle. Needle gauge and length can depend on the application method for the composition. For example, a syringe without a needle or a larger bore needle can be used to apply the present composition to the annulus fibrosis defect during or following a discectomy or other surgical procedure. In other embodiments, the syringe can be fitted with a hypodermic needle suitable for injection of the composition to the site of the annulus fibrosis defect without surgery, permitting methods of the present technology to provide minimally invasive treatment.

### Uses of Compositions:

The present technology includes uses of compositions for manufacturing medicaments for treating defects in an annulus fibrosis, said composition comprising an angiogenic factor, as described above, and a carrier. Such treatments comprise administering the composition into an annulus fibrosis defect of an intervertebral disc.

In some embodiments, administering the composition to the annulus fibrosis defect includes applying the composition during a surgical procedure, such as a discectomy. For example, the composition may be injected into the annulus fibrosis at the site of the defect during surgery. The present methods can be in lieu of spine fusion surgery or implantation of partial or fully artificial discs.

Without limitation to the scope, function or utility of this technology, in various embodiments, administration of compositions of the present disclosure can promote angiogenesis at a site. Treatment of an annulus fibrosis defect using the present methods can heal, promote healing, or provide more complete healing of the defect. Openings in the annulus fibrosis leading to the nucleus pulposus are subsequently sealed, preventing further extravasation of the disk and/or leaking of nucleus pulposus material thereby reducing pain and/or the probability of reherniation through the discectomy hole. Application or delivery of the present compositions may result in more complete vascularization and/or re-vascularization of the defect site.

The following non-limiting example illustrates the compositions, methods, and processes of the present technology.

### Example

A patient exhibiting degenerative disc disease between the fifth lumbar and the top of the sacrum is treated using a composition of the present invention. A transverse tear in the patient's annulus fibrosis has resulted in release of some nucleus pulposus material, including inflammatory cytokines, stimulating nerve roots and causing pain in the patient.

A syringe containing the synthetic molecule F2A4-K-NS and a hyaluronic acid carrier is used to inject the composition into the annulus fibrosis of the defective disc. The composition promotes angiogenesis where new blood vessels form to supply oxygenated blood and nutrients to the site and also allow for waste removal during the healing process. The treatment prevents further degeneration of the disc by regeneration and closure of the annulus fibrosis tear. Consequently, the patient does not require spinal fusion or artificial disc replacement surgery.

In the above example, collagen, heparin sulfate or propylene glycol is substituted for hyaluronic acid, to form gel carriers, with substantially similar results.

The examples and other embodiments described herein are exemplary and not intended to be limiting in describing the full scope of compositions and methods of this technology. Equivalent changes, modifications and variations of specific embodiments, materials, compositions and methods may be made within the scope of the present technology, with substantially similar results.

## Claims

1. The use of a composition for the manufacture of a medicament for the treatment of an annulus fibrosis defect, said composition comprising
(a) an angiogenic factor having the formula: 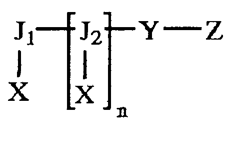
wherein each X represents a synthetic peptide chain that binds an FGF receptor and (i) has a minimum of three amino acid residues, (ii) has a maximum of about fifty amino acid residues, and (iii) binds an FGF receptor;
n is 0 or 1;
J¹ represents an amino acid;
J² represents a diamino acid when n=1, or is absent when n=0;
Y represents a linker that (i) comprises a chain of a minimum of about nine and a maximum of about fifty atoms, (ii) is not found in the natural ligand of the FGF receptor, and (iii) is covalently bonded to J¹ and Z when n=0, or to J² and Z when n=1;
Z represents a non-signaling peptide that comprises a heparin binding domain, comprising an amino acid sequence that comprises (i) a minimum of one heparin binding motif, (ii) a maximum of about ten heparin binding motifs, and (iii) a maximum of about thirty amino acids;
wherein when n=1 the synthetic peptide chains, X are identical; and
wherein the peptide analog has an avidity for heparin such that the synthetic heparin-binding growth factor analog binds heparin in 0.48M NaCl, but is eluted by 1M NaCl; and
(b) a carrier.

2. The use according to Claim 1, wherein X comprises an amino acid sequence found in any of FGF-1, FGF-2, FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, and FGF-23.

3. The use according to Claim 1, wherein the angiogenic factor is F2A4-K-NS.

4. The use according to Claim 1, wherein said carrier is a gel.

5. The use according to Claim 1, wherein the carrier comprises a material selected from the group consisting of collagen, keratin, chitosan, laminin, fibronectin, vitronectin, poly-D-lysine, agarose, cellulose, polyethylene glycol, polypropylene glycol, poly(2-acrylamido-2-methyl-1-propanesulfonic acid), polyvinyl pyrrolidone, silicone hydrogel, glycoaminoglycans, polyacrylamide, and mixtures thereof.

6. The use according to Claim 5, wherein the carrier comprises a material is a glycosaminoglycan selected from the group consisting of chondroitin sulphate, dermatan sulphate, keratan sulphate, heparan sulphate, heparin, and hyaluronic acid.

7. The use according to Claim 1, wherein the administering step comprises injecting the composition at the annulus fibrosis defect.

8. The use according to Claim 1, wherein the annulus fibrosis defect comprises an opening resulting from a discectomy.

9. A syringe containing an injectable composition, the composition comprising:
(a) an angiogenic factor having the formula: 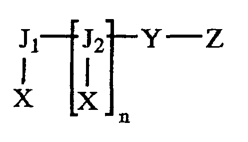
wherein each X represents a synthetic peptide chain that binds an FGF receptor and (i) has a minimum of three amino acid residues, (ii) has a maximum of about fifty amino acid residues, and (iii) binds an FGF receptor;
n is 0 or 1;
J¹ represents an amino acid;
J² represents a diamino acid when n=1, or is absent when n=0;
Y represents a linker that (i) comprises a chain of a minimum of about nine and a maximum of about fifty atoms, (ii) is not found in the natural ligand of the FGF receptor, and (iii) is covalently bonded to J¹ and Z when n=0, or to J² and Z when n=1;
Z represents a non-signaling peptide that comprises a heparin binding domain, comprising an amino acid sequence that comprises (i) a minimum of one heparin binding motif, (ii) a maximum of about ten heparin binding motifs, and (iii) a maximum of about thirty amino acids;
wherein when n=1 the synthetic peptide chains, X are identical; and
wherein the peptide analog has an avidity for heparin such that the synthetic heparin-binding growth factor analog binds heparin in 0.48M NaCl, but is eluted by 1M NaCl; and
(b) a gel carrier.

10. A syringe according to Claim 9, wherein X comprises an amino acid sequence found in any of FGF-1, FGF-2, FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, and FGF-23.

11. A syringe according to Claim 9, wherein the angiogenic factor is F2A4-K-NS.

12. A syringe according to Claim 9, wherein the carrier is selected from the group consisting of collagen, keratin, chitosan, laminin, fibronectin, vitronectin, poly-D-lysine, agarose, cellulose, polyethylene glycol, polypropylene glycol, poly(2-acrylamido-2-methyl-1-propanesulfonic acid), polyvinyl pyrrolidone, silicone hydrogel, glycosaminoglycans, polyacrylamide, and mixtures thereof.

13. A syringe according to Claim 12, wherein the carrier is a glycosaminoglycan selected from the group consisting of chondroitin sulphate, dermatan sulphate, keratan sulphate, heparan sulphate, heparin, and hyaluronic acid.

14. A composition for treating an annulus defect, said composition comprising:
(a) an angiogenic factor having the formula: 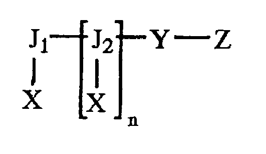
wherein each X represents a synthetic peptide chain that binds an FGF receptor and (i) has a minimum of three amino acid residues, (ii) has a maximum of about fifty amino acid residues, and (iii) binds an FGF receptor;
n is 0 or 1;
J¹ represents an amino acid;
J² represents a diamino acid when n=1, or is absent when n=0;
Y represents a linker that (i) comprises a chain of a minimum of about nine and a maximum of about fifty atoms, (ii) is not found in the natural ligand of the FGF receptor, and (iii) is covalently bonded to J¹ and Z when n=0, or to J² and Z when n=1;
Z represents a non-signaling peptide that comprises a heparin binding domain, comprising an amino acid sequence that comprises (i) a minimum of one heparin binding motif, (ii) a maximum of about ten heparin binding motifs, and (iii) a maximum of about thirty amino acids;
wherein when n=1 the synthetic peptide chains, X are identical; and
wherein the peptide analog has an avidity for heparin such that the synthetic heparin-binding growth factor analog binds heparin in 0.48M NaCl, but is eluted by 1M NaCl; and
(b) a carrier.

15. A composition according to Claim 14, wherein X comprises an amino acid sequence found in any of FGF-1, FGF-2, FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, and FGF-23.

16. A composition according to Claim 14, wherein the angiogenic factor is F2A4-K-NS.

17. A composition according to Claim 14, wherein said carrier is a gel.

18. A composition according to Claim 14, wherein the carrier comprises a material selected from the group consisting of collagen, keratin, chitosan, laminin, fibronectin, vitronectin, poly-D-lysine, agarose, cellulose, polyethylene glycol, polypropylene glycol, poly(2-acrylamido-2-methyl-1-propanesulfonic acid), polyvinyl pyrrolidone, silicone hydrogel, glycoaminoglycans, polyacrylamide, and mixtures thereof.

19. A composition according to Claim 14, wherein the carrier comprises a material is a glycosaminoglycan selected from the group consisting of chondroitin sulphate, dermatan sulphate, keratan sulphate, heparan sulphate, heparin, and hyaluronic acid.
